# EUROPEAN PATENT SPECIFICATION

(11) **EP 3 500 306 B1**
(45) Date of publication and mention of the grant of the patent: **08.07.2020**
(21) Application number: 17764658.5
(22) Date of filing: 14.08.2017
(51) Int. Cl.: A61K 45/06, A61K 31/13, A61K 31/27, A61K 31/445, A61K 31/5377, A61K 31/55, A61P 25/28

(54) **TRIPLE COMBINATION OF HISTAMINE-3 RECEPTOR INVERSE AGONISTS, ACETYLCHOLINESTERASE INHIBITORS AND NMDA RECEPTOR ANTAGONIST**
DREIFACHKOMBINATION AUS INVERSEN HISTAMIN-3-REZEPTORAGONISTEN, ACETYLCHOLINESTERASE-INHIBITOREN UND NMDA-REZEPTORANTAGONIST
TRIPLE COMBINAISON D'AGONISTES INVERSES DU RÉCEPTEUR D'HISTAMINE-3, D'INHIBITEURS DE L'ACÉTYLCHOLINESTÉRASE ET D'ANTAGONISTES DU RÉCEPTEURS NMDA

(30) Priority: 18.08.2016 IN 201641028165
(43) Date of publication of application: 26.06.2019
(73) Proprietor: Suven Life Sciences Limited, Hyderabad, Telangana 500034 (IN)
(72) Inventor: NIROGI, Ramakrishna, Hyderabad Telangana 500034 (IN); SHINDE, Anil Karbhari, Hyderabad Telangana 500034 (IN); MOHAMMED, Abdul Rasheed, Hyderabad Telangana 500034 (IN); JAYARAJAN, Pradeep, Hyderabad Telangana 500034 (IN); BHYRAPUNENI, Gopinadh, Hyderabad Telangana 500034 (IN); JASTI, Venkateswarlu, Hyderabad Telangana 500034 (IN)
(74) Representative: HGF
(86) International application number: PCT/IB2017/054939
(87) International publication number: WO 2018/033848

(56) References cited:
- WO-A1-2014/136075
- US-A1- 2014 135 304
- ZLOMUZICA ARMIN ET AL: "Neuronal histamine and cognitive symptoms in Alzheimer's disease", NEUROPHARMACOLOGY, PERGAMON PRESS, OXFORD, GB, vol. 106, 27 May 2015 (2015-05-27), pages 135-145, XP029599393, ISSN: 0028-3908, DOI: 10.1016/J.NEUROPHARM.2015.05.007
- KHAN NADIA ET AL: "The dual-acting H3 receptor antagonist and AChE inhibitor UW-MD-71 dose-dependently enhances memory retrieval and reverses dizocilpine-induced memory impairment in rats", BEHAVIOURAL BRAIN RESEARCH, ELSEVIER, AMSTERDAM, NL, vol. 297, 20 October 2015 (2015-10-20), pages 155-164, XP029331734, ISSN: 0166-4328, DOI: 10.1016/J.BBR.2015.10.022

## Description

### FIELD OF THE INVENTION

The present invention relates to histamine-3 receptor (H₃R) inverse agonists or the pharmaceutically acceptable salt(s) thereof in combination with or as adjunct to acetylcholinesterase inhibitors (AChEIs) and N-Methyl-D-aspartate (NMDA) receptor antagonist. The present invention further relates to the use of the combination and the pharmaceutical composition containing the said combination in the treatment of cognitive disorders.

### BACKGROUND OF INVENTION

Alzheimer's disease (AD) is the most common cause of dementia worldwide. The exponential rise in the number of cases of AD in the past and the future projection over the next few decades is anticipated to result in great pressure on the social and health-care systems of developed and developing economies alike. AD also imposes tremendous emotional and financial burden to the patient's family and community.

The current list of approved cognitive enhancing drugs for AD is not long and historically been focused on acetylcholinesterase inhibitors (donepezil, rivastigmine and galantamine). These drugs act by inhibiting the hydrolysis of acetylcholine (ACh) into acetate and choline by targeting acetylcholinesterase (AChE) enzyme. Increasing the ACh levels in the synapse can stimulate cholinergic receptors and promote memory function. Although acetylcholinesterase inhibitors (AChEIs) can temporarily delay the progression of cognitive decline in AD, their effects are modest. ACh being present both in the central and peripheral nervous system, AChEIs produce several undesirable side effects such as gastrointestinal disturbances, bradycardia and excessive salivation that are associated with an action on peripheral muscarinic cholinergic receptors (Expert Opinion on Drug Safety, 3, 2004, 425-440). The limitation of AChE inhibitor class of drugs is that they are poorly tolerated, their efficacy is not sustained and they require constant dose-titration as the disease progresses (Cochrane Database Systematic Reviews, 2006, CD005593) which lead to significant patient noncompliance. The incidence and the severity of these side effects increase with the dose amount and in general more pronounced at the initiation of the treatment or after dose increase. Hence there is an unmet need of alternate therapy for treating cognition disorders.

The H₃R is a G protein-coupled receptor (GPCR), mainly expressed in the anterior part of the cortex, hippocampus and the striatum. H₃Rs function as both autoreceptors and heteroreceptors. It modulates the synthesis and release of several neurotransmitters which play an important role in cognition, mood and sensory gating. Preliminary literature reports suggest that H₃R antagonists may have promising utility for the treatment of various CNS disorders including AD, schizophrenia, attention-deficit hyperactivity disorder (ADHD), epilepsy, narcolepsy, neuropathic pain and metabolic disorders. Antagonism of this receptor by several investigational compounds has been shown to improve learning and memory in animal models.

Since blocking H₃R modulates histaminergic and cholinergic activity, one might expect H₃R inverse agonist to complement and/or augment cognitive function of AChEIs. This may in turn help to reduce side effects with better patient compliance and thus can be administered over a long period.

The glutamatergic system is also involved in learning and memory and is a target for treatment of Alzheimer's disease. Memantine, another approved treatment for Alzheimer's disease, acts on the glutamatergic system by inhibiting NMDA receptor under conditions of excess stimulation. It may act to protect glutamate neurons from excessive glutamate stimulation, while increasing the signal to noise ratio. It is known that glutamate neurons have synaptic connections on cholinergic neurons in brain areas associated with learning and memory.

Since the cause and development of the dementia depend on the different mechanisms, it may be an advantageous to use the combination of drugs working in different mechanism for the treatment of AD. The current approved treatment for AD includes the combination of acetylcholinesterase inhibitor, donepezil and NMDA receptor antagonist, memantine. However, there remains the need for the new drugs/combination to treat the patients with AD.

The compounds of the present invention are H₃R inverse agonists with high affinity and very high selectivity over closely related receptor subtypes and improves learning and memory in animals. The H₃R inverse agonist compounds mentioned here are described in US9079888B2. The preparation of these compounds is given in the said patent.

As the treatment of AD is chronic in nature, there is a desperate unmet medical need for better and safer treatment options. A therapeutic strategy eagerly sought for AD patients is to target an improvement with an adjunct to existing therapies that would bring additional relief for patients, lower the burden on the caregiver and allow the patient to enjoy a better quality of life without the need for institutional care and/or hospitalization.

Zlomuzica et al. ("Neuronal histamine and cognitive symptoms in Alzheimer's disease", Neuropharmacology, 2016, 106, p 135-145) discusses the significance of the neuronal histaminergic system as a promising target for the development of more effective drugs for the treatment of cognitive symptoms, and further relates to the use of histamine-related agents as neurogenesis-stimulating therapy that counteracts progressive brain atrophy in Alzheimer's disease.

WO 2014/136075 relates to histamine H₃ receptor subtype selective ligands, pharmaceutical compositions containing such compounds, the use of such compounds as medicaments for the treatment and/or prevention of conditions which require modulation of H₃ receptors, and combination of such compounds and an acetylcholinesterase inhibitor.

Khan et al. ("The dual-acting H3 receptor antagonist and AChE inhibitor UW-MD-71 dose-dependently enhances memory retrieval and reverses dizocilpine-induced memory impairment in rats", Behavioural Brain Research, 2016, 297, p 155-164) discloses dual-active histamine H₃ receptor antagonists and acetylcholinesterase inhibitors for use in the potential treatment of cognitive disorders like Alzheimer's disease.

US 2014/135304 relates to novel compounds and compositions which are useful in the treatment of various disorders that are related to histamine H₃ receptors.

The instant invention provides H₃R inverse agonist compounds or the pharmaceutically acceptable salt(s) thereof, which enhances the cognitive function of patients on treatment in combination with AChEIs and NMDA receptor antagonist. The present invention is based on the unusual finding that the combination of compounds with H₃R inverse agonist activity, the compounds which act as AChEIs (for example donepezil) and the compounds which act as NMDA receptor antagonists (for example memantine), demonstrate synergistic effect in their pharmacological activity. Memantine acts by blocking the glutamatergic neurotransmissions in the brain when the levels are excessively high. Histamine modulates the release of glutamate from corticostriatal nerve terminals. Hence it is not anticipated that the combination of a H₃R inverse agonist + donepezil + memantine would result in synergistic pro-cognitive effects. However surprisingly, the combination of H₃R inverse agonist + AChEIs + NMDA receptor antagonist (triple combination) showed synergistic effects in animal models and also increased the levels acetylcholine, the neurotransmitter which plays a vital role in cognitive improvement. Based on these results one can infer that such combined administration and/or co-treatment of H₃R inverse agonist + AChEIs + NMDA receptor antagonist, may result in beneficial effect to

improve the therapeutic efficacy in humans. Further the H₃R inverse agonist compounds or the pharmaceutically acceptable salt(s) thereof of the instant invention enhances the effect of the AChEIs and NMDA receptor antagonist in the treatment of cognitive disorders.

### SUMMARY OF THE INVENTION

The objective of the present invention is to provide an improved combination therapy for the treatment of cognitive disorders, such as Alzheimer's disease, schizophrenia, Parkinson's disease, lewy body dementia, vascular dementia, frontotemporal dementia, Down syndrome or Tourette's syndrome.

In the first aspect, the present invention relates to a combination comprising a histamine-3 receptor inverse agonist, an acetylcholinesterase inhibitor and a NMDA receptor antagonist; wherein the histamine-3 receptor inverse agonist is selected from:
N-[4-(1-Cyclobutylpiperidin-4-yloxy)phenyl]-2-(morpholin-4-yl)acetamide;
N-[4-(1-Cyclopropylpiperidin-4-yloxy)phenyl]-2-(morpholin-4-yl)acetamide; and
N-[4-(1-Isopropylpiperidin-4-yloxy)phenyl]-2-(morpholin-4-yl)acetamide; or a pharmaceutically acceptable salt thereof.

In embodiments, the histamine-3 receptor inverse agonist is N-[4-(1-Cyclobutylpiperidin-4-yloxy)phenyl]-2-(morpholin-4-yl)acetamide or a pharmaceutically acceptable salt thereof.

In embodiments, the histamine-3 receptor inverse agonist is N-[4-(1-Cyclopropylpiperidin-4-yloxy)phenyl]-2-(morpholin-4-yl)acetamide or a pharmaceutically acceptable salt thereof.

In embodiments, the histamine-3 receptor inverse agonist is N-[4-(1-Isopropylpiperidin-4-yloxy)phenyl]-2-(morpholin-4-yl)acetamide or a pharmaceutically acceptable salt thereof.

In embodiments, the acetylcholinesterase inhibitor is selected from donepezil, galantamine and rivastigmine or a pharmaceutically acceptable salt thereof.

In embodiments, the NMDA receptor antagonist is memantine or a pharmaceutically acceptable salt thereof.

In embodiments, the present invention relates to a combination of N-[4-(1-Cyclobutylpiperidin-4-yloxy)phenyl]-2-(morpholin-4-yl)acetamide, donepezil and memantine or a pharmaceutically acceptable salt thereof.

In embodiments, the present invention relates to the combination of N-[4-(1-Cyclopropylpiperidin-4-yloxy)phenyl]-2-(morpholin-4-yl)acetamide, donepezil and memantine or a pharmaceutically acceptable salt thereof.

In embodiments, the present invention relates to the combination of N-[4-(1-Isopropylpiperidin-4-yloxy)phenyl]-2-(morpholin-4-yl)acetamide, donepezil and memantine or a pharmaceutically acceptable salt thereof.

In another aspect, the present invention relates to the said combination for use in the treatment of cognitive disorders in a patient, preferably wherein the cognitive disorder is selected from Alzheimer's disease, schizophrenia, Parkinson's disease, Lewy body dementia, vascular dementia, frontotemporal dementia, Down syndrome or Tourette's syndrome.

In yet another aspect, the present invention relates to the compound, N-[4-(1-Cyclobutylpiperidin-4-yloxy)phenyl]-2-(morpholin-4-yl)acetamide or a pharmaceutically acceptable salt thereof for use in combination with or adjunct to an acetylcholinesterase inhibitor and NMDA receptor antagonist for the treatment of Alzheimer's disease in a patient, and preferably wherein the use is an adjunct treatment for Alzheimer's disease in a patient on stable treatment with acetylcholinesterase inhibitor and NMDA receptor antagonist.

In another aspect, the present invention relates to pharmaceutical composition comprising the combination of the invention, and pharmaceutically acceptable excipients or combination thereof.

In another aspect, the present invention relates to pharmaceutical composition comprising the combination of the invention, and the pharmaceutically acceptable excipients or combination thereof for use in the treatment of cognitive disorders selected from Alzheimer's disease, schizophrenia, Parkinson's disease, Lewy body dementia, vascular dementia, and frontotemporal dementia.

### BRIEF DESCRIPTION OF ACCOMPANYING DRAWINGS

Figure 1a depicts the results of the effect of a co-treatment of compound 1 with donepezil and memantine on cognition enhancing properties using object recognition task model.
Figure 1b depicts the results of the effect of a co-treatment of compound 3 with donepezil and memantine on cognition enhancing properties using object recognition task model.
Figure 2 depicts the effect of compound 1 in combination with donepezil and memantine on extracellular levels of acetylcholine in medial prefrontal cortex of male Wistar rats.
Figure 3 depicts the effect of compound 2 in combination with donepezil and memantine on extracellular levels of acetylcholine in medial prefrontal cortex of male Wistar rats.
Figure 4 depicts the effect of compound 3 in combination with donepezil and memantine on extracellular levels of acetylcholine in medial prefrontal cortex of male Wistar rats.
Figure 5 depicts the effect of compound 1 in combination with donepezil and memantine on evoked theta levels in dorsal hippocampus of anesthetized male Wistar rats.

### DETAILED DESCRIPTION OF THE INVENTION

Unless otherwise stated, the following terms used in the specification and claims have the meanings given below:
The term, "histamine-3 receptor inverse agonist" as used herein refers to a ligand or drug that binds with the constitutively active histamine-3 receptors, stabilize them and thus reduce the activity (negative intrinsic activity). It blocks or inhibits the function / binding of agonist at the H₃ receptor and exert opposite pharmacological effect of a receptor agonist.

Examples of the histamine-3 receptor inverse agonist include, N-[4-(1-Cyclobutylpiperidin-4-yloxy)phenyl]-2-(morpholin-4-yl)acetamide; N-[4-(1-Cyclopropylpiperidin-4-yloxy)phenyl]-2-(morpholin-4-yl)acetamide; and N-[4-(1-Isopropylpiperidin-4-yloxy)phenyl]-2-(morpholin-4-yl)acetamide; or a pharmaceutically acceptable salt thereof.

Examples of pharmaceutically acceptable salt of the above identified compounds include but not limited to,
N-[4-(1-Cyclobutylpiperidin-4-yloxy)phenyl]-2-(morpholin-4-yl)acetamide dihydrochloride; N-[4-(1-Cyclopropylpiperidin-4-yloxy)phenyl]-2-(morpholin-4-yl)acetamide tartrate; and
N-[4-(1-Isopropylpiperidin-4-yloxy)phenyl]-2-(morpholin-4-yl)acetamide tartrate.

The term, "acetylcholinesterase inhibitor" as used herein is a chemical or drug that inhibits the acetylcholinesterase enzyme from breaking down acetylcholine, thereby increasing both the level and duration of action of the neurotransmitter acetylcholine. Examples of acetylcholinesterase inhibitor are donepezil, rivastigmine and galantamine. Preferably, the acetylcholinesterase inhibitor is donepezil and rivastigmine. More preferably the acetylcholinesterase inhibitor is donepezil.

Donepezil is a drug approved for treatment of mild, moderate and severe dementia of Alzheimer's disease. Donepezil is a reversible inhibitor of the enzyme acetylcholinesterase and sold under trade name Aricept® as hydrochloride salt.

Rivastigmine is a drug approved for treatment of mild, moderate and severe dementia of Alzheimer's disease. Rivastigmine is a reversible cholinesterase inhibitor and sold under trade name Exelon® and Exelon Patch® as tartrate salt.

Galantamine is a drug approved for treatment of mild, moderate and severe dementia of Alzheimer's disease. Galantamine, a reversible, competitive acetylcholinesterase inhibitor and sold under trade name Razadyne® as hydrobromide salt.

The term, "NMDA receptor antagonist" as used herein refers to class of compounds which act on glutamatergic system by inhibiting the NMDA receptor. Example of NMDA receptor antagonist is memantine. Memantine is a drug approved for treatment of moderate to severe dementia of the Alzheimer's disease. Memantine is NMDA receptor antagonist and sold under trade name Namenda® and Namenda XR® as hydrochloride salt.

The combination of memantine and donepezil is approved for the treatment of moderate to severe dementia of the Alzheimer's disease and sold under trade name Namzaric™ as memantine hydrochloride salt and donepezil hydrochloride salt.

The phrase, "therapeutically effective amount" is defined as an amount of a compound of the present invention that (i) treats the particular disease, condition or disorder, (ii) eliminates one or more symptoms of the particular disease, condition or disorder and (iii) delays the onset of one or more symptoms of the particular disease, condition or disorder described herein.

The term, "pharmaceutically acceptable salt" as used herein refers to salts of the active compound and are prepared by reaction with the appropriate organic or inorganic acid or acid derivative, depending on the particular substituents found on the compounds described herein.

The term, "patient" as used herein refers to an animal. Preferably the term "patient" refers to mammal. The term mammal includes animals such as mice, rats, dogs, rabbits, pigs, monkeys, horses and human. More preferably the patient is human.

The term, "Alzheimer's disease" as used herein refers to a dementia that causes problems with memory, thinking and behavior. The Alzheimer's disease can be mild to moderate to severe Alzheimer's disease.

The compound 1 as used herein is N-[4-(1-Cyclobutylpiperidin-4-yloxy)phenyl]-2-(morpholin-4-yl)acetamide dihydrochloride which has the chemical structure,

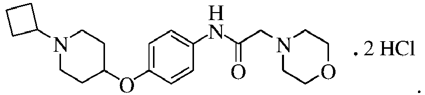

The compound 2 as used herein is N-[4-(1-Cyclopropylpiperidin-4-yloxy)phenyl]-2-(morpholin-4-yl)acetamide tartrate which has the chemical structure,

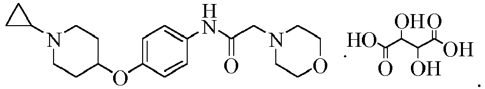

The compound 3 as used herein is N-[4-(1-Isopropylpiperidin-4-yloxy)phenyl]-2-(morpholin-4-yl)acetamide tartrate which has the chemical structure,

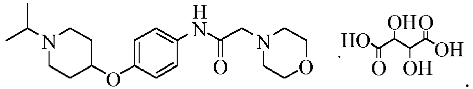

The term, "treatment' or 'treating" as used herein refers to any treatment of a disease in a mammal, including: (a) slowing or arresting the development of clinical symptoms; and/or (b) causing the regression of clinical symptoms.

The term, "compound for use" as used herein embrace any one or more of the following: (1) use of a compound, (2) method of use of a compound, (3) use in the treatment of, or (4) the use for the manufacture of pharmaceutical composition / medicament for treatment / treating.

The term, "cognitive disorder" as used herein refers to a group of mental health disorders that principally affect learning, memory, perception and problem solving and includes amnesia, dementia and delirium. Cognitive disorders can result due to disease, disorder, ailment or toxicity. Example of cognitive disorders includes but not limited to, Alzheimer's disease, schizophrenia, Parkinson's disease, lewy body dementia (LBD), vascular dementia, frontotemporal dementia (FTD), Down syndrome or Tourette's syndrome. Preferably, the cognitive disorder is Alzheimer's disease.

The term, "adjunct" or "adjunctive treatment" as used herein refers to an additional treatment to a patient who has already received at least one other therapy for cognitive disorders. A drug used as adjunctive therapy is administered to a patient to make that primary treatment works better.

### Embodiments

The present invention encompasses all the combinations described herein without limitation, however, preferred aspects and elements of the invention are discussed herein in the form of the following embodiments.

In one embodiment, the present invention relates to the combination of histamine-3 receptor inverse agonist, acetylcholinesterase inhibitor and NMDA receptor antagonist; wherein the histamine-3 receptor inverse agonist is N-[4-(1-Cyclobutylpiperidin-4-yloxy)phenyl]-2-(morpholin-4-yl)acetamide dihydrochloride.

In another embodiment, the present invention relates to the combination of histamine-3 receptor inverse agonist, acetylcholinesterase inhibitor and NMDA receptor antagonist; wherein the histamine-3 receptor inverse agonist is N-[4-(1-Cyclopropylpiperidin-4-yloxy)phenyl]-2-(morpholin-4-yl)acetamide tartrate.

In another embodiment, the present invention relates to the combination of histamine-3 receptor inverse agonist, acetylcholinesterase inhibitor and NMDA receptor antagonist; wherein the histamine-3 receptor inverse agonist is N-[4-(1-Isopropylpiperidin-4-yloxy)phenyl]-2-(morpholin-4-yl)acetamide tartrate.

In another embodiment, the present invention relates to the combination of N-[4-(1-Cyclobutylpiperidin-4-yloxy)phenyl]-2-(morpholin-4-yl)acetamide, rivastigmine and memantine or a pharmaceutically acceptable salt thereof.

In another embodiment, the present invention relates to the combination of N-[4-(1-Cyclobutylpiperidin-4-yloxy)phenyl]-2-(morpholin-4-yl)acetamide, galantamine and memantine or a pharmaceutically acceptable salt thereof.

In another embodiment, the present invention relates to the combination of N-[4-(1-Cyclopropylpiperidin-4-yloxy)phenyl]-2-(morpholin-4-yl)acetamide, rivastigmine and memantine or a pharmaceutically acceptable salt thereof.

In another embodiment, the present invention relates to the combination of N-[4-(1-Cyclopropylpiperidin-4-yloxy)phenyl]-2-(morpholin-4-yl)acetamide, galantamine and memantine or a pharmaceutically acceptable salt thereof.

In another embodiment, the present invention relates to the combination of N-[4-(1-Isopropylpiperidin-4-yloxy)phenyl]-2-(morpholin-4-yl)acetamide, rivastigmine and memantine or a pharmaceutically acceptable salt thereof.

In another embodiment, the present invention relates to the combination of N-[4-(1-Isopropylpiperidin-4-yloxy)phenyl]-2-(morpholin-4-yl)acetamide, galantamine and memantine or a pharmaceutically acceptable salt thereof.

In another embodiment, the present invention relates to the combination of N-[4-(1-Cyclobutylpiperidin-4-yloxy)phenyl]-2-(morpholin-4-yl)acetamide dihydrochloride, donepezil hydrochloride and memantine hydrochloride.

In another embodiment, the present invention relates to the combination of N-[4-(1-Cyclobutylpiperidin-4-yloxy)phenyl]-2-(morpholin-4-yl)acetamide dihydrochloride, rivastigmine tartrate and memantine hydrochloride.

In another embodiment, the present invention relates to the combination of N-[4-(1-Cyclobutylpiperidin-4-yloxy)phenyl]-2-(morpholin-4-yl)acetamide dihydrochloride, galantamine hydrobromide and memantine hydrochloride.

In another embodiment, the present invention relates to the combination of N-[4-(1-Cyclopropylpiperidin-4-yloxy)phenyl]-2-(morpholin-4-yl)acetamide tartrate, donepezil hydrochloride and memantine hydrochloride.

In another embodiment, the present invention relates to the combination of N-[4-(1-Cyclopropylpiperidin-4-yloxy)phenyl]-2-(morpholin-4-yl)acetamide tartrate, rivastigmine tartrate and memantine hydrochloride.

In another embodiment, the present invention relates to the combination of N-[4-(1-Cyclopropylpiperidin-4-yloxy)phenyl]-2-(morpholin-4-yl)acetamide tartrate, galantamine hydrobromide and memantine hydrochloride.

In another embodiment, the present invention relates to the combination of N-[4-(1-Isopropylpiperidin-4-yloxy)phenyl]-2-(morpholin-4-yl)acetamide tartrate, donepezil hydrochloride and memantine hydrochloride.

In another embodiment, the present invention relates to the combination of N-[4-(1-Isopropylpiperidin-4-yloxy)phenyl]-2-(morpholin-4-yl)acetamide tartrate, rivastigmine tartrate and memantine hydrochloride.

In another embodiment, the present invention relates to the combination of N-[4-(1-Isopropylpiperidin-4-yloxy)phenyl]-2-(morpholin-4-yl)acetamide tartrate, galantamine hydrobromide and memantine hydrochloride.

In another embodiment, the present invention provides the combination of histamine-3 receptor inverse agonist, acetylcholinesterase inhibitor and NMDA receptor antagonist which is more effective than the combination of histamine-3 receptor inverse agonist and acetylcholinesterase inhibitor, acetylcholinesterase inhibitor and NMDA receptor antagonist or histamine-3 receptor inverse agonist and NMDA receptor antagonist.

In another embodiment, the present invention provides the combination of the histamine-3 receptor inverse agonist, acetylcholinesterase inhibitor and NMDA receptor antagonist which is more effective than the histamine-3 receptor inverse agonist, acetylcholinesterase inhibitor and NMDA receptor antagonist alone.

In another embodiment, the present invention provides the combination of N-[4-(1-Cyclobutylpiperidin-4-yloxy)phenyl]-2-(morpholin-4-yl)acetamide dihydrochloride, donepezil hydrochloride and memantine hydrochloride which is more effective than the combination of N-[4-(1-Cyclobutylpiperidin-4-yloxy)phenyl]-2-(morpholin-4-yl)acetamide dihydrochloride and donepezil hydrochloride, donepezil hydrochloride and memantine hydrochloride or N-[4-(1-Cyclobutylpiperidin-4-yloxy)phenyl]-2-(morpholin-4-yl)acetamide dihydrochloride and memantine hydrochloride.

In another embodiment, the present invention provides the combination of N-[4-(1-Cyclobutylpiperidin-4-yloxy)phenyl]-2-(morpholin-4-yl)acetamide dihydrochloride, donepezil hydrochloride and memantine hydrochloride which is more effective than N-[4-(1-Cyclobutylpiperidin-4-yloxy)phenyl]-2-(morpholin-4-yl)acetamide dihydrochloride, donepezil hydrochloride and memantine hydrochloride alone.

In another embodiment the pharmaceutically acceptable salt of histamine-3 receptor inverse agonist includes but not limited to, dihydrochloride salt, oxalate salt, succinate, tartrate salt and the like. Preferably, the pharmaceutically acceptable salt is dihydrochloride salt and tartrate salts. More preferably, the pharmaceutically acceptable salt is dihydrochloride salt.

In another variant, the present disclosure relates to a method of treating Alzheimer's disease comprising administering to a patient in need thereof a therapeutically effective amount of the said combination.

In another variant, the present disclosure relates to a method of treating Alzheimer's disease comprising administering to a patient in need thereof a therapeutically effective amount of N-[4-(1-Cyclobutylpiperidin-4-yloxy)phenyl]-2-(morpholin-4-yl)acetamide or a pharmaceutically acceptable salt thereof, acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclopropylpiperidin-4-yloxy)phenyl]-2-(morpholin-4-yl)acetamide or a pharmaceutically acceptable salt thereof, acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Isopropylpiperidin-4-yloxy)phenyl]-2-(morpholin-4-yl)acetamide or a pharmaceutically acceptable salt thereof, acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclobutylpiperidin-4-yloxy)phenyl]-2-(morpholin-4-yl)acetamide or a pharmaceutically acceptable salt thereof in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclopropylpiperidin-4-yloxy)phenyl]-2-(morpholin-4-yl)acetamide or a pharmaceutically acceptable salt thereof in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Isopropylpiperidin-4-yloxy)phenyl]-2-(morpholin-4-yl)acetamide or a pharmaceutically acceptable salt thereof in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclobutylpiperidin-4-yloxy)phenyl]-2-(morpholin-4-yl)acetamide dihydrochloride in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclopropylpiperidin-4-yloxy)phenyl]-2-(morpholin-4-yl)acetamide tartrate in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Isopropylpiperidin-4-yloxy)phenyl]-2-(morpholin-4-yl)acetamide tartrate in combination with acetylcholinesterase inhibitor and NMDA receptor antagonist.

In another variant, the present disclosure relates to a method of treating Alzheimer's disease comprising administering to a patient in need thereof a therapeutically effective amount of N-[4-(1-Cyclobutylpiperidin-4-yloxy)phenyl]-2-(morpholin-4-yl)acetamide dihydrochloride in combination with donepezil or a pharmaceutically acceptable salt thereof and memantine or a pharmaceutically acceptable salt thereof.

In another variant, the present disclosure relates to a method of treating Alzheimer's disease comprising administering to a patient in need thereof a therapeutically effective amount of N-[4-(1-Cyclobutylpiperidin-4-yloxy)phenyl]-2-(morpholin-4-yl)acetamide dihydrochloride in combination with donepezil hydrochloride and memantine hydrochloride.

In another embodiment, the present invention relates to the combination of N-[4-(1-Cyclobutylpiperidin-4-yloxy)phenyl]-2-(morpholin-4-yl)acetamide dihydrochloride, acetylcholinesterase inhibitor and NMDA receptor antagonist for use in the treatment of Alzheimer's disease.

In yet another aspect, the present invention relates to N-[4-(1-Cyclobutylpiperidin-4-yloxy)phenyl]-2-(morpholin-4-yl)acetamide or a pharmaceutically acceptable salt thereof for use in combination with acetylcholinesterase inhibitor and NMDA receptor antagonist in the adjunct treatment of Alzheimer's disease in a patient on treatment with acetylcholinesterase inhibitor and NMDA receptor antagonist.

In yet another variant, the present disclosure relates to N-[4-(1-Cyclopropylpiperidin-4-yloxy)phenyl]-2-(morpholin-4-yl)acetamide or a pharmaceutically acceptable salt thereof for use in the adjunct treatment of Alzheimer's disease in a patient on treatment with acetylcholinesterase inhibitor and NMDA receptor antagonist.

In yet another variant, the present disclosure relates to N-[4-(1-Isopropylpiperidin-4-yloxy)phenyl]-2-(morpholin-4-yl)acetamide or a pharmaceutically acceptable salt thereof for use in the adjunct treatment of Alzheimer's disease in a patient on treatment with acetylcholinesterase inhibitor and NMDA receptor antagonist.

In another embodiment, the present invention relates to N-[4-(1-Cyclobutylpiperidin-4-yloxy)phenyl]-2-(morpholin-4-yl)acetamide dihydrochloride for use in combination with acetylcholinesterase inhibitor and NMDA receptor antagonist in the adjunct treatment of Alzheimer's disease in a patient on treatment with donepezil and memantine or a pharmaceutically acceptable salt thereof.

In another variant, the present disclosure relates to N-[4-(1-Cyclopropylpiperidin-4-yloxy)phenyl]-2-(morpholin-4-yl)acetamide tartrate for use in the adjunct treatment of Alzheimer's disease in a patient on treatment with donepezil and memantine or a pharmaceutically acceptable salt thereof.

In another variant, the present disclosure relates to N-[4-(1-Isopropyl piperidin-4-yloxy)phenyl]-2-(morpholin-4-yl)acetamide tartrate for use in the adjunct treatment of Alzheimer's disease in a patient on treatment with donepezil and memantine or a pharmaceutically acceptable salt thereof.

In another variant, the present disclosure relates to use of the combination of histamine-3 receptor inverse agonist, acetylcholinesterase inhibitor and NMDA receptor antagonist in the manufacture of a medicament for treatment of Alzheimer's disease.

In another variant, the present disclosure relates to use of histamine-3 receptor inverse agonist in the manufacture of a medicament for treatment of Alzheimer's disease in combination with acetylcholinesterase inhibitor and NMDA receptor antagonist.

In another variant, the present disclosure relates to use of histamine-3 receptor inverse agonist in the manufacture of a medicament for treatment of Alzheimer's disease as adjunct to acetylcholinesterase inhibitor and NMDA receptor antagonist.

In another variant, the present disclosure relates to use of the N-[4-(1-Cyclobutylpiperidin-4-yloxy)phenyl]-2-(morpholin-4-yl)acetamide or a pharmaceutically acceptable salt thereof in the manufacture of a medicament for the treatment of Alzheimer's disease in combination with donepezil or a pharmaceutically acceptable salt thereof and memantine or a pharmaceutically acceptable salt thereof.

In another variant, the present disclosure relates to use of the N-[4-(1-Cyclobutylpiperidin-4-yloxy)phenyl]-2-(morpholin-4-yl)acetamide dihydrochloride in the manufacture of a medicament for treatment of Alzheimer's disease in combination with donepezil or a pharmaceutically acceptable salt thereof and memantine or a pharmaceutically acceptable salt thereof.

In another variant, the present disclosure relates to use of N-[4-(1-Cyclobutylpiperidin-4-yloxy)phenyl]-2-(morpholin-4-yl)acetamide dihydrochloride in the manufacture of a medicament for treatment of Alzheimer's disease in combination with donepezil hydrochloride and memantine hydrochloride.

In another variant, the present disclosure relates to histamine-3 receptor inverse agonist for use in the treatment of Alzheimer's disease in combination with Namzaric™.

In another variant, the present disclosure relates to a method of treating Alzheimer's disease comprising administering to a patient in need thereof a therapeutically effective amount of histamine-3 receptor inverse agonist in combination with Namzaric™.

In another embodiment, the present invention relates to the combination of the invention for treatment of Alzheimer's disease, wherein Alzheimer's disease is mild Alzheimer's disease.

In another embodiment, the present invention relates to the combination of the invention for treatment of Alzheimer's disease, wherein the Alzheimer's disease is moderate Alzheimer's disease.

In another embodiment, the present invention relates to the combination of the invention for treatment of Alzheimer's disease, wherein the Alzheimer's disease is severe Alzheimer's disease.

In another embodiment, the present invention relates to the combination of the invention wherein the active ingredients can be administered to a patient concurrently or separately.

In yet another embodiment, the active ingredients of the combination of the present invention are normally administered by formulating the active ingredients into a pharmaceutical composition in accordance with standard pharmaceutical practice.

In yet another embodiment, the active ingredients of the combination of the present invention may be administered by oral, nasal, local, dermal or parenteral routes.

In yet another embodiment, the active ingredients of the combination of the present invention can be administered by the same or different route of administration. For instance, the histamine-3 receptor inverse agonist of the instant invention can be administered orally, the acetylcholinesterase inhibitor can be administered transdermally and the NMDA receptor antagonist can be administered locally.

The pharmaceutical compositions of the present invention may be formulated in a conventional manner using one or more pharmaceutically acceptable excipients. The pharmaceutically acceptable excipients are diluents, disintegrants, binders, lubricants, glidants, polymers, coating agents, solvents, cosolvents, preservatives, wetting agents, thickening agents, antifoaming agents, sweetening agents, flavouring agents, antioxidants, colorants, solubilizers, plasticizer, dispersing agents and the like. Excipients are selected from microcrystalline cellulose, mannitol, lactose, pregelatinized starch, sodium starch glycolate, corn starch or derivatives thereof, povidone, crospovidone, calcium stearate, glyceryl monostearate, glyceryl palmitostearate, talc, colloidal silicone dioxide, magnesium stearate, sodium lauryl sulfate, sodium stearyl fumarate, zinc stearate, stearic acid or hydrogenated vegetable oil, gum arabica, magnesia, glucose, fats, waxes, natural or hardened oils, water, physiological sodium chloride solution or alcohols, for example, ethanol, propanol or glycerol, sugar solutions, such as glucose solutions or mannitol solutions and the like or a mixture of the various excipients.

In yet another embodiment, the active compounds of the invention may be formulated in the form of pills, tablets, coated tablets, capsules, powder, granules, pellets, patches, implants, films, liquids, semi-solids, gels, aerosols, emulsions, elixirs and the like. Such pharmaceutical compositions and processes for preparing same are well known in the art.

In yet another embodiment, the pharmaceutical composition of the instant invention contains 1 to 90 %, 5 to 75 % and 10 to 60 % by weight of the compounds of the instant invention or pharmaceutically acceptable salt thereof. The amount of the active compounds or its pharmaceutically acceptable salt in the pharmaceutical composition(s) can range from about 0.1 mg to about 100 mg or from about 0.1 mg to about 60 mg or from about 0.1 mg to about 30 mg or in any range falling within the broader range of 0.1 mg to 100 mg.

In yet another embodiment, the pharmaceutical composition of the combination of the instant invention can be conventional formulations such as immediate release formulations, modified release formulations such as sustained release formulations, delayed release formulations and extended release formulations or new delivery systems such as oral disintegrating formulations and transdermal patches.

The dose of the active compounds can vary depending on factors such as age and weight of patient, nature, route of administration and severity of the disease to be treated and such other factors. Therefore, any reference regarding pharmacologically effective amount of the compounds 1, 2 and 3 refers to the aforementioned factors.

In yet another embodiment, the histamine-3 receptor inverse agonist can be co-administered with acetylcholinesterase inhibitor and NMDA receptor antagonist at a daily dose of 0.1 mg to 100 mg; such as 0.1, 0.5, 0.75, 1, 1.5, 3, 5, 6, 10, 20, 25, 30, 50, 75 and 100 mg, preferably at a daily dose of 0.1, 3, 5, 6, 10, 20, 25, 30 or 50 mg and most preferably at a daily dose of 0.5, 3, 5, 10 or 20 mg.

In yet another embodiment, the acetylcholinesterase inhibitor can be co-administered with N histamine-3 receptor inverse agonist and NMDA receptor antagonist at a daily dose of 1 mg to 30 mg; such as 1, 1.5, 2, 3, 4, 4.5, 5, 6, 8, 9.5,10, 12, 13, 13.3, 15, 16, 23, 24, 25 or 30 mg, preferably at a daily dose of 1, 1.5, 2, 3, 4, 4.5, 5, 6, 8, 9.5, 10, 12, 13, 13.3, 16, 23, 24, or 25 mg and most preferably at a daily dose of 1.5, 3, 4, 4.5, 5, 6, 8, 9.5, 10, 12, 13.3, 16, 23 or 24 mg.

In yet another embodiment, the NMDA receptor antagonist, memantine can be co-administered with histamine-3 receptor inverse agonist and acetylcholinesterase inhibitor at a daily dose of 1 mg to 40 mg; such as 5, 7, 10, 14, 20, 28 or 40 mg, preferably at a daily dose of 5, 7, 10, 14, 20 or 28 mg and most preferably at a daily dose of 5, 10, 14, 20 or 28 mg.

In yet another embodiment, the acetylcholinesterase inhibitor, donepezil can be co-administered with histamine-3 receptor inverse agonist and NMDA receptor antagonist at a daily dose of 2 mg to 30 mg; such as 2, 5, 10, 15, 23, 25 or 30 mg, preferably at a daily dose of 2, 5, 10, 23 or 25 mg and most preferably at a daily dose of 5, 10 or 23 mg.

In yet another embodiment, the acetylcholinesterase inhibitor, rivastigmine can be co-administered with histamine-3 receptor inverse agonist and NMDA receptor antagonist at a daily dose of 0.5 mg to 15 mg; such as 1, 1.5, 3, 4.5, 5, 6, 9.5, 10 or 13.3 mg, preferably at a daily dose of 1, 1.5, 3, 4.5, 5, 6, 9.5 or 13.3 mg and most preferably at a daily dose of 1.5, 3, 4.5, 6, 9.5 and 13.3 mg.

In yet another embodiment, the acetylcholinesterase inhibitor, galantamine can be co-administered with histamine-3 receptor inverse agonist and NMDA receptor antagonist at a daily dose of 1 mg to 30 mg; such as 1, 2, 4, 6, 8, 12, 16, 24 and 30 mg, preferably at a daily dose of 2, 4, 6, 8, 12, 16 and 24 mg and most preferably at a daily dose of 4, 8, 12, 16 and 24 mg.

In yet another embodiment, the treatment comprises administering to the patient 0.1 mg to 100 mg of N-[4-(1-Cyclobutylpiperidin-4-yloxy)phenyl]-2-(morpholin-4-yl)acetamide or a pharmaceutically acceptable salt thereof, per day.

In yet another embodiment, the treatment comprises administering to the patient 0.1 mg to 60 mg of N-[4-(1-Cyclobutylpiperidin-4-yloxy)phenyl]-2-(morpholin-4-yl)acetamide or a pharmaceutically acceptable salt thereof, per day.

In yet another embodiment, the treatment comprises administering to the patient 0.1 mg to 30 mg of N-[4-(1-Cyclobutylpiperidin-4-yloxy)phenyl]-2-(morpholin-4-yl)acetamide or a pharmaceutically acceptable salt thereof, per day.

In yet another embodiment, the treatment comprises administering to the patient 1 mg to 25 mg of donepezil or a pharmaceutically acceptable salt thereof, per day.

In yet another embodiment, the treatment comprises administering to the patient 5 mg to 25 mg of donepezil or a pharmaceutically acceptable salt thereof, per day.

In yet another embodiment, the treatment comprises administering to the patient, 5, 10 or 23 mg of donepezil or a pharmaceutically acceptable salt thereof, per day.

In yet another embodiment, the treatment comprises administering to the patient 1 mg to 40 mg of memantine or a pharmaceutically acceptable salt thereof, per day.

In yet another embodiment, the treatment comprises administering to the patient 5 mg to 30 mg of memantine or a pharmaceutically acceptable salt thereof, per day.

In yet another embodiment, the treatment comprises administering to the patient 5, 10, 14, 20 or 28 mg of memantine or a pharmaceutically acceptable salt thereof, per day.

In yet another embodiment, the treatment comprises administering the active compounds to the patient one to three times per day, one to three times per week or one to three times per month. Preferably, the treatment comprises administering the compound to a patient once a day, twice a day, or thrice a day. More preferably, the treatment comprises administering the compound to a patient once a day.

### Examples

The examples given below are provided by the way of illustration only and therefore should not be construed to limit the scope of the invention.

### Abbreviations:

- ANOVA: : Analysis of variance
- AP: : Anterior Posterior
- aCSF: : Artificial Cerebrospinal fluid
- CaCl₂. 2H₂O: : Calcium chloride dihydrate
- DV: : Dorsal Ventral
- DTT: : Dithiothreitol
- EC₅₀: : Half maximal effective concentration
- EDTA: : Ethylenediaminetetraacetic acid
- EEG: : Electroencephalogram
- GDP: : Guanosine diphosphate
- GPCR: : G-Protein Coupled Receptor
- HCl: : Hydrochloric acid
- h: : Hour (s)
- HEPES: : 4-(2-Hydroxyethyl)piperazine-1-ethanesulfonic acid
- *i.p.*: : Intraperitoneal
- *i.v.*: : Intravenous
- KCl: : Potassium chloride
- K_{b}: : Binding constant
- Kᵢ: : Inhibitory constant
- LC-MS/MS: : Liquid chromatography-Mass spectrometry/ Mass spectrometry
- mg: : Milligram
- MgCl₂: : Magnesium chloride
- min: : Minute (s)
- ML: : Medial Lateral
- mM: : Millimolar
- nmol/L: : Nanomoles per litre
- NaCl: : Sodium chloride
- NaH₂PO₄.2H₂O: : Sodium dihydrogen phosphate dihydrate
- Na₂HPO₄.7H₂O: : Sodium monohydrogen phosphate heptahydrate
- NPO: : Nucleus Pontis Oralis
- nM: : Nanomolar
- *p.o.*: : Per oral
- *s.c.*: : Subcutaneous
- S.E.M.: : Standard error of the mean
- µM: : Micromolar
- θ: : Theta

### Example 1: Determination of Kᵢ value at human and rat histamine-3 receptor

Test compounds were evaluated according to the following procedures to determine the Kᵢ value at human and rat histamine-3 receptor.

### Materials and Methods:

Receptor source: Rat brain frontal cortex or recombinant human cDNA expressed in CHO cells
Radioligand: [3H] R-α-methylhistamine
Final ligand concentration: [3.0 nM]
Non-specific determinant: R-α-methylhistamine (100 µM)
Reference compound: R-α-methylhistamine
Positive control: R-α-methylhistamine

### Incubation conditions:

Increasing concentrations of test compounds or standard were incubated with membrane receptors and radioligand in 5 mM MgCl₂ and 50 mM TRIS-HCl (pH 7.4) for 60 minutes at room temperature. The reaction was terminated by rapid vacuum filtration onto the glass fiber filters. Radioactivity trapped onto the filters was determined and compared to the control values in order to ascertain any interactions of the test compound(s) with either cloned human or rat receptor binding site.

### Results:

| S. No | Example | Human histamine-3 receptor Kᵢ (nM) | Rat histamine-3 receptor Kᵢ (nM) |
|---|---|---|---|
| 1 | Compound 1 | 8.7 | 9.8 |
| 2 | Compound 2 | 19.9 | ND |
| 3 | Compound 3 | 8.3 | ND |

| | | | |
|---|---|---|---|
| ND-Not done | | | |

### Reference:

Br J Pharmacol., 2008, 154(6): 1166-1181.

### Example 2: Determination of IC₅₀ values at histamine-3 receptor

Test compounds were evaluated according to the following procedures to determine the IC₅₀ values.

### Materials and Methods:

| | |
|---|---|
| Receptor source: | Human recombinant (CHO-K1 cells) |
| Radioligand: | [35S]-GTPγS |
| Final ligand concentration: | [0.3 nM] |
| Reference compound: | Thioperamide |
| Positive control: | Thioperamide |

### Incubation conditions:

Increasing concentrations of test compounds and/or vehicle is preincubated with the membranes (0.09 mg/mL) and 10 µM GDP in modified HEPES pH 7.4 buffer (20 mM HEPES, pH 7.4, 100 mM NaCl, 10 mM MgCl₂, 1 mM DTT, 1 mM EDTA) for 20 minutes and SPA beads are then added for another 60 minutes at 30°C. The reaction is initiated by 0.3 nM [³⁵S]GTPγS for an additional 30 minutes incubation period. Test compound-induced increase of [³⁵S]GTPγS binding by 50 percent or more (≥50 %) relative to the 3 µM R(-)-a-methylhistamine response indicates possible histamine-3 receptor agonist activity. Test compound induced inhibition of 0.03 µM R(-)-a-methylhistamine-induced increase of [³⁵S]GTPγS binding response by 50 percent or more (≥50 %) indicates receptor antagonist activity. These studies were conducted and the data were analyzed at Eurofins Panlabs Taiwan Ltd, Taiwan using standard radioligand binding techniques as described above.

### Results:

Compound 1 exhibits inverse agonist like properties in GTPyS assay on human recombinant histamine-3 receptor with IC₅₀ value of 20 nM.

### Reference:

J. Neurochem., 1998, 71(2): 808-816.

### Example 3: Object Recognition Task Model

The cognition enhancing properties of compounds of this invention were estimated by using this model.

Male Wistar rats (8-10 weeks old) were used as experimental animals. Four animals were housed in each cage. Animals were kept on 20 % food deprivation from a day prior to experimentation. Water was provided ad libitum throughout the experiment. Animals were maintained on a 12 hours light/dark cycle in temperature and humidity controlled room. The experiment was carried out in an open field made up of acrylic. Rats were habituated to individual arenas (open field) in the absence of any objects on day 1.

Rats received vehicle, donepezil and memantine or test compound, donepezil and memantine on the day of habituation, before familiar (T₁) and choice (T₂) trials. During the familiarization phase (T₁), the rats were placed individually in the arena for 3 minutes, in which two identical objects (a₁ and a₂) were positioned 10 cm from the wall. 24 hours after T₁, trial for long-term memory test was assessed. The same rats were placed in the same arena as they were placed in T₁ trial. During the choice phase (T₂) rats were allowed to explore the arena for 3 minutes in presence of a copy of familiar object (a₃) and one novel object (b). During the T₁ and T₂ trial, explorations of each object (defined as sniffing, licking, chewing or having moving vibrissae whilst directing the nose towards the object at a distance of less than 1 cm) were recorded using stopwatch.
T₁ is the total time spent exploring the familiar objects (a₁ + a₂).
T₂ is the total time spent exploring the familiar object and novel object (a₃ +b).

Discriminative index is ratio of time spent exploring the novel object divided by sum of time spent exploring the novel object and familiar object in choice trial (T₂).

The object recognition test was performed as described in Behavioural Brain Research, 1988, 31, 47-59.

### Results:

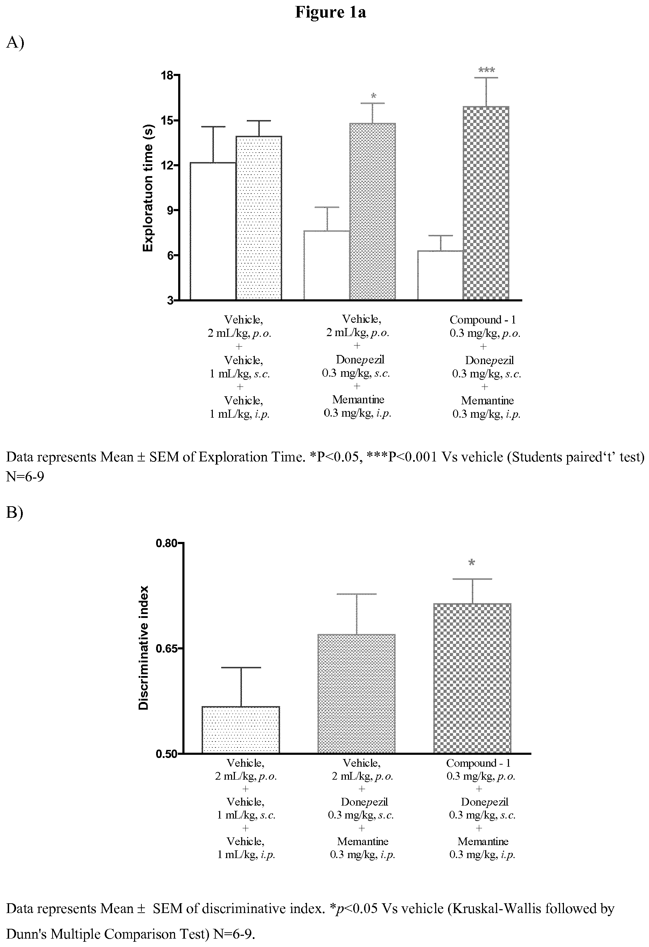

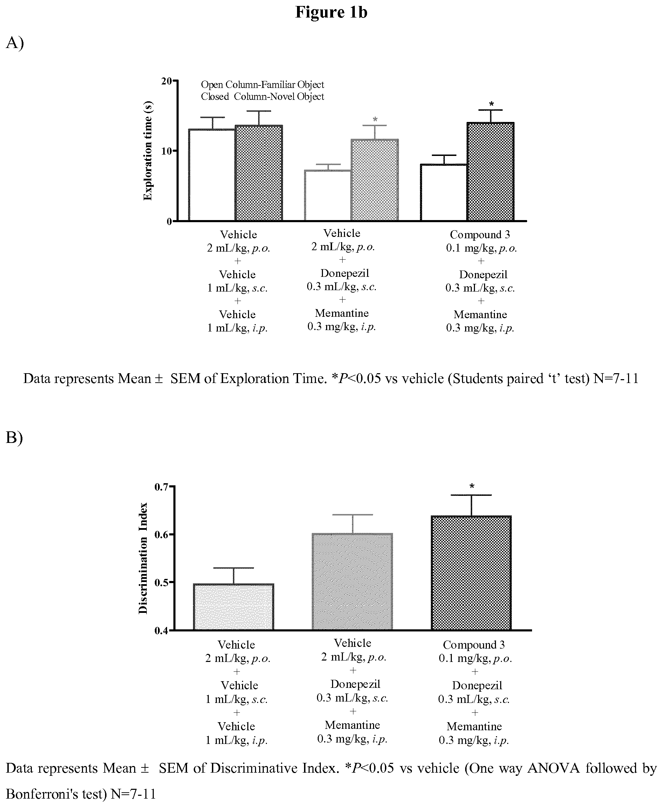

Vehicle treated animals spent almost equal time exploring the novel and the familiar objects. The groups treated with combination of test compound, donepezil and memantine spent significantly more time exploring the novel object. No significant increase in discriminative index was observed in the group treated with donepezil and memantine when compared to the vehicle treatment. However the group co-treated with test compounds, donepezil and memantine showed significant improvement in the memory end point (discriminative index). This procognitive effect suggests a potentiating effect of test compounds over the procognitive effect of donepezil and memantine. The results of this study are provided in figure 1a and 1b.

### Example 4: Evaluation of acetylcholine modulation in medial prefrontal cortex of male Wistar rats

Neurotransmitter modulating effects of triple combination were evaluated by this model.

Male Wistar rats (240-300 g body weight) were stereotaxically implanted with a microdialysis guide cannula in medial prefrontal cortex (mPFC; AP: +3.2 mm, ML: -0.5 mm, DV: -3.0 mm) under isoflurane anesthesia. Co-ordinates were taken according to atlas for the rat brain (Paxinos and Watson, 2004) with reference points taken from bregma and vertical from the skull. The rats were allowed to recover individually for four days in a round bottom Plexiglas bowl with free access to feed and water.

After surgical recovery of 4 days, male Wistar rats were connected to dual quartz lined two-channel liquid swivel (Instech, UK) on a counter balance lever arm, which allowed unrestricted movements of the animal. Sixteen hours before start of the study, a pre-equilibrated microdialysis probe (2 mm dialysis membrane) was inserted into mPFC through the guide cannula. On the day of study, probe was perfused with artificial cerebrospinal fluid (aCSF; NaCl 147 mM, KCl 2.7 mM, MgCl₂ 1 mM, CaCl₂. 2H₂O 1.2 mM, pH 7.4) at a flow rate of 1.5 µL/min and a stabilization period of 2 hours was maintained. Five basal samples were collected at 20 min intervals prior to the treatment of test compounds (3 or 10 mg/kg, *p.o.*) or vehicle. Donepezil (1 mg/kg, *s.c.*) and memantine (1 mg/kg, *s.c.*) were administered 30 min after administration of test compounds. Dialysate samples were collected for an additional period of 4 h post treatment of test compounds. Dialysates were stored below -50°C prior to analysis.

### Quantitation of acetylcholine:

Acetylcholine concentrations in dialysates were quantified using LC-MS/MS based method.

### Statistical analysis:

All microdialysis data for acetylcholine was plotted as percent change from mean dialysate basal concentrations with 100 % defined as the average of five pre-dose values. The percent change in acetylcholine levels were compared with donepezil and memantine combination using two-way analysis of variance (time and treatment), followed by Bonferroni's posttest. Area under the curve (AUC) values for percent change in acetylcholine levels was calculated and the statistical significance between the mean AUC values was compared against donepezil and memantine treatment using unpaired "t" test. Statistical significance was considered at a *p* value less than 0.05. Incorrect probe placement was considered as criteria to reject the data from animal.

### Reference:

1. Paxinos G. and Watson C. (2004) Rat brain in stereotaxic coordinates. Academic Press, New York.

### Results:

### Compound 1

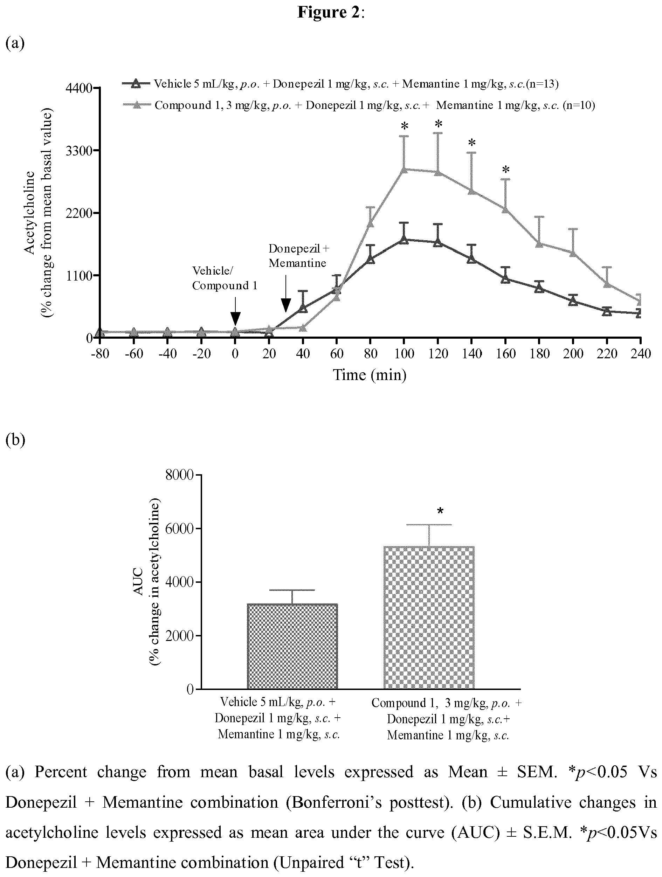

Treatment with donepezil and memantine produced increase in acetylcholine levels to the maximum of 1726 ± 297 % of basal levels. The increase in acetylcholine after combination of compound 1, donepezil and memantine was significantly higher compared to donepezil and memantine combination. Mean maximum increase in acetylcholine was observed to be 2968 ± 585 of pre-dose levels after triple combination (Figure 2(a)).

Mean area under the curve values (AUC) calculated after the treatment of compound 1, donepezil and memantine were significantly higher compared to donepezil and memantine combination (Figure 2(b)).

### Compound 2

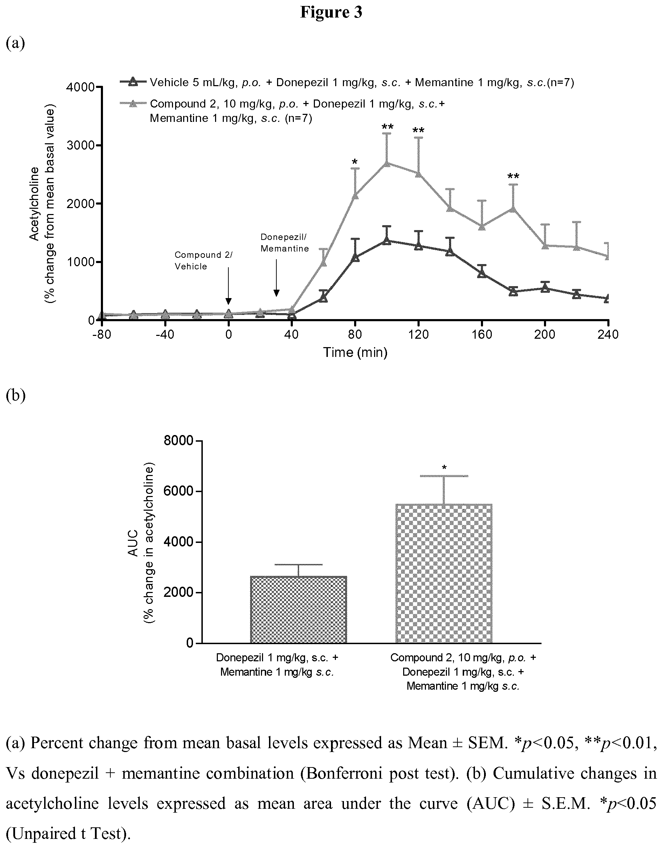

Treatment with donepezil and memantine produced increase in acetylcholine levels to the maximum of 1365 ± 249 % of basal levels. The increase in acetylcholine after combination of compound 2, donepezil and memantine was significantly higher compared to donepezil and memantine combination. Mean maximum increase in acetylcholine was observed to be 2696 ± 504 % of pre-dose levels after triple combination (Figure 3(a)).

Mean area under the curve values (AUC) calculated after treatment of compound 2, donepezil and memantine were significantly higher compared to donepezil and memantine combination (Figure 3(b)).

### Compound 3

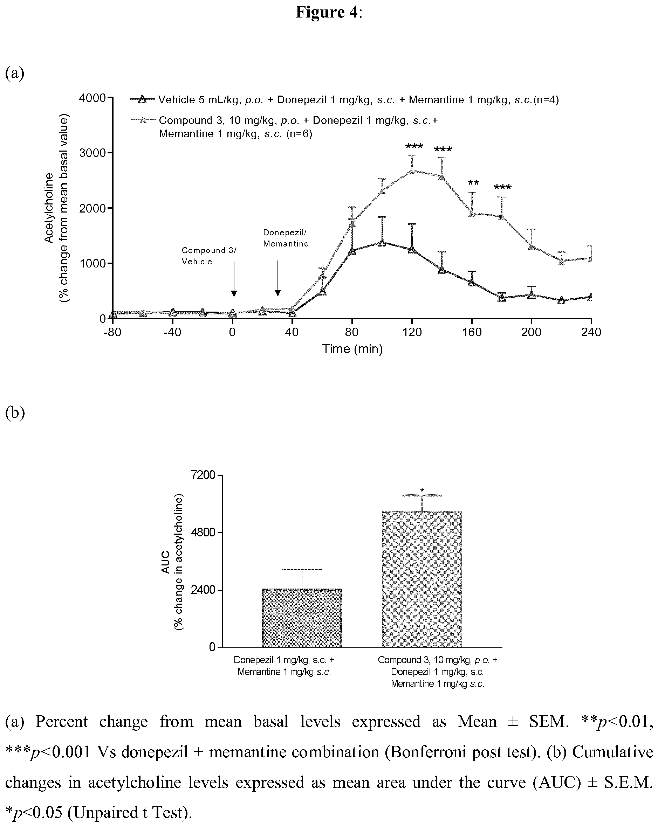

Treatment with donepezil and memantine produced increase in acetylcholine levels to the maximum of 1375 ± 461 % of basal levels. The increase in acetylcholine after combination of compound 3, donepezil and memantine was significantly higher compared to donepezil and memantine combination. Mean maximum increase in acetylcholine was observed to be 2674 ±271 of pre-dose levels after triple combination (Figure 4(a)).

Mean area under the curve values (AUC) calculated after treatment of compound 3, donepezil and memantine were significantly higher compared to donepezil and memantine combination (Figure 4(b)).

### Example 5: Evaluation of theta modulation in dorsal hippocampus of anesthetized male Wistar rats

Effect of triple combination on brain activity as a pharmacodynamic endpoint is evaluated using this model.

Male Wistar rats (240-320 g) were anesthetized by intraperitoneal administration of urethane (1.2 to 1.5 g/kg) for implantation of a catheter in the left femoral vein. The animal was placed in a stereotaxic frame for implanting an electrode (stainless steel wire, Plastics One) into the dorsal hippocampus (AP: - 3.8 mm; ML: +2.2 mm; DV: -2.5 mm; Paxinos and Watson, 2004). Bipolar stimulating electrode (untwisted stainless steel wires, separated by 0.75-1.0 mm at their tips, Plastics One) was implanted in the Nucleus Pontis Oralis (NPO; AP: - 7.8 mm; ML: 1.8 mm; DV: -6.0 mm; Paxinos and Watson, 2004). Additionally one electrode was implanted into the cerebellum which served as a reference. Hippocampal θ rhythm was evoked via a 6-s electrical stimulation train (20-160 µA, 0.3-ms pulse duration, 250 Hz) delivered to the NPO at a rate of 0.01 trains/s with a Grass S88 stimulator and PSIU6 stimulus isolation unit (Grass Medical Instruments, Quincy, MA). EEG was recorded at a rate of 1000 Hz using Ponemah (Version 5.2) software and stored for off-line analysis using NeuroScore (Version 3.0). Baseline amplitude level was achieved by using the current required to elicit θ rhythm to 50% of the maximal amplitude under control conditions. After the stabilization period of one hour, baseline recording was done for 30 min followed by the treatment of vehicle or compound 1 (1 mg/kg, i.v.). Donepezil (0.3 mg/kg, *i.v.*) and memantine (0.3 mg/kg, *i.v.*) was administered 30 min after compound 1 treatment and recording was continued for additional 1 hour.

### Statistical analysis:

Power in the θ rhythm frequency in the stimulation period during the 30-min baseline period was calculated and the % changes in these measures post treatment were calculated. The percent change in relative theta power after triple combination of Compound 1, donepezil and memantine was compared with donepezil and memantine using two-way analysis of variance (time and treatment), followed by Bonferroni's posttest. Statistical significance was considered at a *p* value less than 0.05.

### Reference:

1. Paxinos G. and Watson C. (2004) Rat brain in stereotaxic coordinates. Academic Press, New York.

### Results:

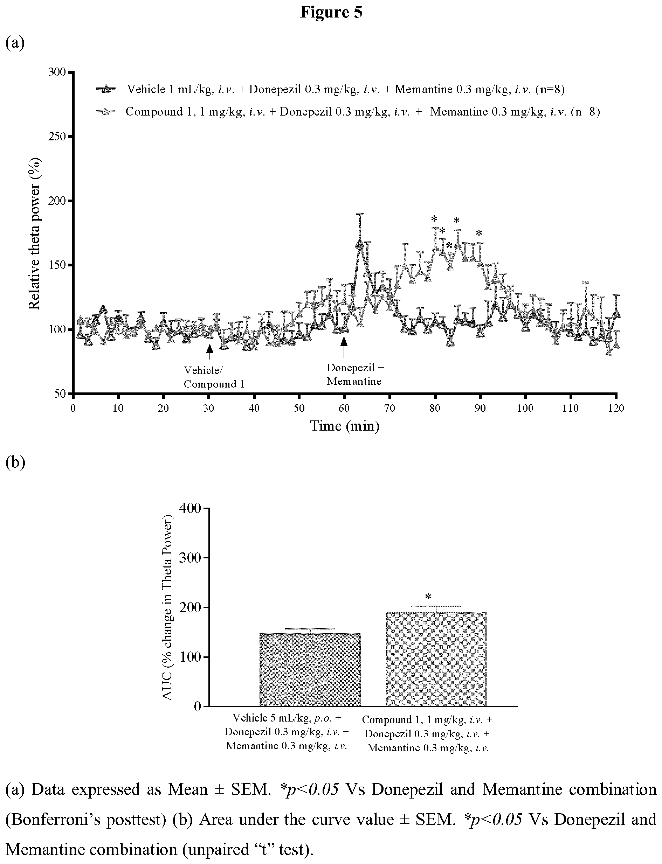

Treatment with donepezil and memantine combination produced moderate increase in hippocampal θ power. Compound 1 in combination with donepezil and memantine produced significant increase in θ power levels and peak levels reached up to 167 ± 11 % of pre-dose levels. The effect in triple combination was observed to be significantly higher than the combination of donepezil and memantine (Figure 5(a)).

Mean area under the curve values (AUC) calculated after the treatment of compound 1, donepezil and memantine were significantly higher compared to donepezil and memantine combination (Figure 5(b)).

## Claims

1. A combination comprising a histamine-3 receptor inverse agonists, an acetylcholinesterase inhibitor and a NMDA receptor antagonist; wherein the histamine-3 receptor inverse agonists is selected from N-[4-(1-Cyclobutylpiperidin-4-yloxy)phenyl]-2-(morpholin-4-yl)acetamide; N-[4-(1-Cyclopropylpiperidin-4-yloxy)phenyl]-2-(morpholin-4-yl)acetamide; and N-[4-(1-Isopropylpiperidin-4-yloxy)phenyl]-2-(morpholin-4-yl)acetamide; or a pharmaceutically acceptable salt thereof.

2. The combination as claimed in claim 1, wherein the acetylcholinesterase inhibitor is selected from the group consisting of donepezil, rivastigmine and galantamine or a pharmaceutically acceptable salt thereof, and preferably wherein the acetylcholinesterase inhibitor is donepezil or a pharmaceutically acceptable salt thereof, more preferably is donepezil hydrochloride.

3. The combination as claimed in claim 1, wherein the NMDA receptor antagonist is memantine or a pharmaceutically acceptable salt thereof and preferably the NMDA receptor antagonist is memantine hydrochloride.

4. The combination as claimed in claim 1, wherein the histamine-3 receptor inverse agonist is N-[4-(1-Cyclobutylpiperidin-4-yloxy)phenyl]-2-(morpholin-4-yl)acetamide or a pharmaceutically acceptable salt thereof, and preferably is N-[4-(1-Cyclopropylpiperidin-4-yloxy)phenyl]-2-(morpholin-4-yl)acetamide N-[4-(1-Cyclobutylpiperidin-4-yloxy)phenyl]-2-(morpholin-4-yl)acetamide dihydrochloride; or N-[4-(1-Cyclopropylpiperidin-4-yloxy)phenyl]-2-(morpholin-4-yl)acetamide tartrate; or wherein the histamine-3 receptor inverse agonist is N-[4-(1-Isopropylpiperidin-4-yloxy)phenyl]-2-(morpholin-4-yl)acetamide tartrate.

5. The combination as claimed in lor 4, wherein the histamine-3 receptor inverse agonist is N-[4-(1-Cyclobutylpiperidin-4-yloxy)phenyl] -2-(morpholin-4-yl)acetamide dihydrochloride.

6. A combination comprising N-[4-(1-Cyclobutylpiperidin-4-yloxy)phenyl]-2-(morpholin-4-yl)acetamide dihydrochloride, donepezil hydrochloride and memantine hydrochloride.

7. The combination as claimed in any one of the claim 1 to 6, for use in the treatment of cognitive disorders in a patient, preferably wherein the cognitive disorder is selected from Alzheimer's disease, schizophrenia, Parkinson's disease, Lewy body dementia, vascular dementia, frontotemporal dementia, Down syndrome and Tourette's syndrome.

8. The compound, N-[4-(1-Cyclobutylpiperidin-4-yloxy)phenyl]-2-(morpholin-4-yl)acetamide or a pharmaceutically acceptable salt thereof for use in combination with acetylcholinesterase inhibitor and NMDA receptor antagonist for the treatment of Alzheimer's disease in a patient, and preferably wherein the use is an adjunct treatment for Alzheimer's disease in a patient on stable treatment with acetylcholinesterase inhibitor and NMDA receptor antagonist.

9. The compound for use as claimed in claim 8, wherein the acetylcholinesterase inhibitor is selected from donepezil, rivastigmine and galantamine or a pharmaceutically acceptable salt thereof.

10. The compound for use as claimed in claim 8, wherein the NMDA receptor antagonist is memantine or a pharmaceutically acceptable salt thereof.

11. The compound for use as claimed in claim 8, wherein the treatment comprises administering to the patient N-[4-(1-Cyclobutylpiperidin-4-yloxy)phenyl]-2-(morpholin-4-yl)acetamide or a pharmaceutically acceptable salt in an amount of 0.1 mg to 100 mg of per day, and preferably in an amount of 0.1 mg to 60 mg per day, and more preferably in an amount of 0.1 mg to 30 mg per day.

12. The compound for use as claimed in claim 8, wherein the treatment comprises administering to the patient 1 mg to 30 mg of donepezil or a pharmaceutically acceptable salt thereof per day.

13. The compound for use as claimed in claim 8, wherein the treatment comprises administering to the patient 1 mg to 40 mg of memantine or a pharmaceutically acceptable salt thereof per day.

14. A pharmaceutical composition comprising the combination as claimed in any one of the claim 1 to 6, and pharmaceutically acceptable excipients or combination thereof.

15. The pharmaceutical composition as claimed in claim 14, for use in the treatment of cognitive disorders selected from Alzheimer's disease, schizophrenia, Parkinson's diseases, Lewy body dementia, vascular dementia and frontotemporal dementia.

16. The pharmaceutical composition as claimed in claim 14, wherein the N- [4-(1-Cyclobutylpiperidin-4-yloxy)phenyl] -2-(morpholin-4-yl)acetamide or a pharmaceutically acceptable salt thereof is present in an amount of 0.1 mg to 100 mg, preferably in an amount of 0.1 mg to 60 mg, and more preferably in an amount of 0.1 mg to 30 mg, and/or , wherein donepezil or a pharmaceutically acceptable salt thereof is present in an amount of 2 mg to 30 mg, and/or wherein mementine of pharmaceutically acceptable salt thereof is present in an amount of 1 mg to 40 mg.

17. The compound for use as claimed in claim 8, wherein the treatment comprises administering N-[4-(1-Cyclobutylpiperidin-4-yloxy)phenyl]-2-(morpholin-4-yl)acetamide or a pharmaceutically acceptable salt thereof to the patient by oral, nasal, local, dermal or parenteral routes.

18. The compound for use as claimed in claim 8, wherein the treatment comprises administering N-[4-(1-Cyclobutylpiperidin-4-yloxy)phenyl]-2-(morpholin-4-yl)acetamide or a pharmaceutically acceptable salt thereof to the patient one to three times per day, one to three times per week or one to three times per month.

## Patentansprüche

1. Kombination, umfassend einen inversen Histamin-3-Rezeptor-Agonisten, einen Acetylcholinesterase-Inhibitor und einen NMDA-Rezeptor-Antagonisten, wobei der inverse Histamin-3-Rezeptor-Agonist ausgewählt ist aus N-[4-(1-Cyclobutylpiperidin-4-yloxy)phenyl]-2-(morpholin-4-yl)acetamid, N-[4-(1-Cyclopropylpiperidin-4-yloxy)phenyl]-2-(morpholin-4-yl)acetamid und N-[4-(1-Isopropylpiperidin-4-yloxy)phenyl]-2-(morpholin-4-yl)acetamid, oder ein pharmazeutisch verträgliches Salz davon.

2. Kombination nach Anspruch 1, wobei der Acetylcholinesterase-Inhibitor aus der Gruppe ausgewählt ist, die aus Donepezil, Rivastigmin und Galantamin oder einem pharmazeutisch verträglichen Salz davon besteht, und wobei der Acetylcholinesterase-Inhibitor vorzugsweise Donepezil oder ein pharmazeutisch verträgliches Salz davon, stärker bevorzugt Donepezilhydrochlorid, ist.

3. Kombination nach Anspruch 1, wobei der NMDA-Rezeptor-Antagonist Memantin oder ein pharmazeutisch verträgliches Salz davon ist und vorzugsweise der NMDA-Rezeptor-Antagonist Memantin-Hydrochlorid ist.

4. Kombination nach Anspruch 1, wobei der inverse Histamin-3-Rezeptor-Agonist N-[4-(1-Cyclobutylpiperidin-4-yloxy)phenyl]-2-(morpholin-4-yl)acetamid oder ein pharmazeutisch verträgliches Salz davon ist und vorzugsweise N-[4-(1-Cyclopropylpiperidin-4-yloxy)phenyl]-2-(morpholin-4-yl)acetamid N-[4-(1-Cyclobutylpiperidin-4-yloxy)phenyl]-2-(morpholin-4-yl)acetamid-dihydrochlorid ist, oder N-[4-(1-Cyclopropylpiperidin-4-yloxy)phenyl]-2-(morpholin-4-yl)acetamidtartrat, oder wobei der inverse Histamin-3-Rezeptor-Agonist N-[4-(1-Isopropylpiperidin-4-yloxy)phenyl]-2-(morpholin-4-yl)acetamidtartrat ist.

5. Kombination, wie in 1 oder 4 beansprucht, wobei der inverse Histamin-3-Rezeptor-Agonist N-[4-(1-Cyclobutylpiperidin-4-yloxy)phenyl]-2-(morpholin-4-yl)acetamiddihydrochlorid ist.

6. Eine Kombination, die N-[4-(1-Cyclobutylpiperidin-4-yloxy)phenyl]-2-(morpholin-4-yl)acetamiddihydrochlorid, Donepezilhydrochlorid und Memantinhydrochlorid umfasst.

7. Kombination nach einem der Ansprüche 1 bis 6 zur Verwendung bei der Behandlung kognitiver Störungen bei einem Patienten, wobei die kognitive Störung vorzugsweise aus Alzheimer-Krankheit, Schizophrenie, Parkinson-Krankheit, Lewy-Körperchen-Demenz, vaskulärer Demenz, frontotemporaler Demenz, Down-Syndrom und Tourette-Syndrom ausgewählt ist.

8. Verbindung, N-[4-(1-Cyclobutylpiperidin-4-yloxy)phenyl]-2-(morpholin-4-yl)acetamid oder ein pharmazeutisch verträgliches Salz davon zur Verwendung in Kombination mit Acetylcholinesterase-Inhibitor und NMDA-Rezeptorantagonist zur Behandlung von Alzheimer-Krankheit bei einem Patienten, und wobei die Verwendung vorzugsweise eine Zusatzbehandlung für die Alzheimer-Krankheit bei einem Patienten bei stabiler Behandlung mit Acetylcholinesterase-Inhibitor und NMDA-Rezeptorantagonist ist.

9. Verbindung zur Verwendung nach Anspruch 8, wobei der Acetylcholinesterase-Inhibitor ausgewählt ist aus Donepezil, Rivastigmin und Galantamin oder einem pharmazeutisch verträglichen Salz davon.

10. Verbindung zur Verwendung nach Anspruch 8, wobei der NMDA-Rezeptor-Antagonist Memantin oder ein pharmazeutisch verträgliches Salz davon ist.

11. Verbindung zur Verwendung nach Anspruch 8, wobei die Behandlung die Verabreichung von N-[4-(1-Cyclobutylpiperidin-4-yloxy)phenyl]-2-(morpholin-4-yl)acetamid oder eines pharmazeutisch verträglichen Salzes an den Patienten in einer Menge von 0,1 mg bis 100 mg pro Tag, und vorzugsweise in einer Menge von 0,1 mg bis 60 mg pro Tag, und stärker bevorzugt in einer Menge von 0,1 mg bis 30 mg pro Tag, umfasst.

12. Verbindung zur Verwendung nach Anspruch 8, wobei die Behandlung die Verabreichung von 1 mg bis 30 mg Donepezil oder eines pharmazeutisch verträglichen Salzes davon pro Tag an den Patienten umfasst.

13. Verbindung zur Verwendung nach Anspruch 8, wobei die Behandlung die Verabreichung von 1 mg bis 40 mg Memantin oder eines pharmazeutisch verträglichen Salzes davon pro Tag an den Patienten umfasst.

14. Eine pharmazeutische Zusammensetzung, umfassend die Kombination, wie in einem der Ansprüche 1 bis 6 beansprucht, und pharmazeutisch verträgliche Hilfsstoffe oder eine Kombination davon.

15. Pharmazeutische Zusammensetzung nach Anspruch 14 zur Verwendung bei der Behandlung kognitiver Störungen, ausgewählt aus Alzheimer-Krankheit, Schizophrenie, Parkinson-Krankheit, Lewy-Körperchen-Demenz, vaskulärer Demenz und frontotemporaler Demenz.

16. Pharmazeutische Zusammensetzung nach Anspruch 14, wobei das N-[4-(1-Cyclobutylpiperidin-4-yloxy)phenyl]-2-(morpholin-4-yl)acetamid oder ein pharmazeutisch verträgliches Salz davon in einer Menge von 0,1 mg bis 100 mg, vorzugsweise in einer Menge von 0,1 mg bis 60 mg, und stärker bevorzugt in einer Menge von 0,1 mg bis 30 mg, vorhanden ist, und/oder wobei Donepezil oder ein pharmazeutisch verträgliches Salz davon in einer Menge von 2 mg bis 30 mg vorhanden ist, und/oder wobei Mementin oder ein pharmazeutisch verträgliches Salz davon in einer Menge von 1 mg bis 40 mg vorhanden ist.

17. Verbindung zur Verwendung nach Anspruch 8, wobei die Behandlung die Verabreichung von N-[4-(1-Cyclobutylpiperidin-4-yloxy)phenyl]-2-(morpholin-4-yl)acetamid oder eines pharmazeutisch verträglichen Salzes davon an den Patienten auf oralem, nasalem, lokalem, dermalem oder parenteralem Weg umfasst.

18. Verbindung zur Verwendung nach Anspruch 8, wobei die Behandlung die Verabreichung von N-[4-(1-Cyclobutylpiperidin-4-yloxy)phenyl]-2-(morpholin-4-yl)acetamid oder eines pharmazeutisch verträglichen Salzes davon an den Patienten ein- bis dreimal pro Tag, ein- bis dreimal pro Woche oder ein- bis dreimal pro Monat umfasst.

## Revendications

1. Combinaison comprenant un agoniste inverse du récepteur de l'histamine 3, un inhibiteur de l'acétylcholinestérase et un antagoniste du récepteur de NMDA ; dans laquelle l'agoniste inverse du récepteur de l'histamine 3 est choisi parmi le N-[4-(1-cyclobutylpipéridin-4-yloxy)phényl]-2-(morpholin-4-yl)acétamide ; le N-[4-(1-cyclopropylpipéridin-4-yloxy)phényl]-2-(morpholin-4-yl)acétamide ; et le N-[4-(1-isopropylpipéridin-4-yloxy)phényl]-2-(morpholin-4-yl)acétamide ; ou un sel de ceux-ci acceptable sur le plan pharmaceutique.

2. Combinaison selon la revendication 1, dans laquelle l'inhibiteur de l'acétylcholinestérase est choisi dans le groupe constitué du donépézil, de la rivastigmine et de la galantamine ou d'un sel de ceux-ci acceptable sur le plan pharmaceutique, et de préférence dans laquelle l'inhibiteur de l'acétylcholinestérase est le donépézil ou un sel de celui-ci acceptable sur le plan pharmaceutique, plus préférablement est le chlorhydrate de donépézil.

3. Combinaison selon la revendication 1, dans laquelle l'antagoniste du récepteur de NMDA est la mémantine ou un sel de celle-ci acceptable sur le plan pharmaceutique et de préférence l'antagoniste du récepteur de NMDA est le chlorhydrate de mémantine.

4. Combinaison selon la revendication 1, dans laquelle l'agoniste inverse du récepteur de l'histamine 3 est le N-[4-(1-cyclobutylpipéridin-4-yloxy)phényl]-2-(morpholin-4-yl)acétamide ou un sel de celui-ci acceptable sur le plan pharmaceutique, et de préférence est le N-[4-(1-cyclopropylpipéridin-4-yloxy)phényl]-2-(morpholin-4-yl)acétamide le dichlorhydrate de N-[4-(1-cyclobutylpipéridin-4-yloxy)phényl]-2-(morpholin-4-yl)acétamide ; ou le tartrate de N-[4-(1-cyclopropylpipéridin-4-yloxy)phényl]-2-(morpholin-4-yl)acétamide ; ou dans laquelle l'agoniste inverse du récepteur de l'histamine 3 est le tartrate de N-[4-(1-isopropylpipéridin-4-yloxy)phényl]-2-(morpholin-4-yl)acétamide.

5. Combinaison selon la revendication 1 ou la revendication 4, dans laquelle l'agoniste inverse du récepteur de l'histamine 3 est le dichlorhydrate de N-[4-(1-cyclobutylpipéridin-4-yloxy)phényl]-2-(morpholin-4-yl)acétamide.

6. Combinaison comprenant le dichlorhydrate de N-[4-(1-cyclobutylpipéridin-4-yloxy)phényl]-2-(morpholin-4-yl)acétamide, le chlorhydrate de donépézil et le chlorhydrate de mémantine.

7. Combinaison selon l'une quelconque des revendications 1 à 6, destinée à être utilisée dans le traitement de troubles cognitifs chez un patient, de préférence dans laquelle le trouble cognitif est choisi parmi la maladie d'Alzheimer, la schizophrénie, la maladie de Parkinson, la démence du corps de Lewy, la démence vasculaire, la démence fronto-temporale, le syndrome de Down et le syndrome de Gilles de la Tourette.

8. Composé, le N-[4-(1-cyclobutylpipéridin-4-yloxy)phényl]-2-(morpholin-4-yl)acétamide ou un sel de celui-ci acceptable sur le plan pharmaceutique destiné à être utilisé en combination avec l'inhibiteur de l'acétylcholinestérase et l'antagoniste du récepteur de NDMA pour le traitement de la maladie d'Alzheimer chez un patient, et de préférence dans lequel l'utilisation est un traitement annexe pour la maladie d'Alzheimer chez un patient sous traitement stable avec l'inhibiteur de l'acétylcholinestérase et l'antagoniste du récepteur de NDMA.

9. Composé destiné à être utilisé selon la revendication 8, dans lequel l'inhibiteur de l'acétylcholinestérase est choisi parmi le donépézil, la rivastigmine et la galantamine ou un sel de ceux-ci acceptable sur le plan pharmaceutique.

10. Composé destiné à être utilisé selon la revendication 8, dans lequel l'antagoniste du récepteur de NMDA est la mémantine ou un sel de celle-ci acceptable sur le plan pharmaceutique.

11. Composé destiné à être utilisé selon la revendication 8, dans lequel le traitement comprend l'administration au patient de N-[4-(1-cyclobutylpipéridin-4-yloxy)phényl]-2-(morpholin-4-yl)acétamide ou d'un sel acceptable sur le plan pharmaceutique en une quantité allant de 0,1 mg à 100 mg par jour, et de préférence en une quantité allant de 0,1 mg à 60 mg par jour, et plus préférablement en une quantité allant de 0,1 mg à 30 mg par jour.

12. Composé destiné à être utilisé selon la revendication 8, dans lequel le traitement comprend l'administration au patient de 1 mg à 30 mg de donépézil ou d'un sel de celui-ci acceptable sur le plan pharmaceutique par jour.

13. Composé destiné à être utilisé selon la revendication 8, dans lequel le traitement comprend l'administration au patient de 1 mg à 40 mg de mémantine ou d'un sel de celle-ci acceptable sur le plan pharmaceutique par jour.

14. Composition pharmaceutique comprenant la combinaison selon l'une quelconque des revendications 1 à 6, et des excipients acceptables sur le plan pharmaceutique ou une combinaison de ceux-ci.

15. Composition pharmaceutique selon la revendication 14, destinée à être utilisée dans le traitement de troubles cognitifs choisis parmi la maladie d'Alzheimer, la schizophrénie, la maladie de Parkinson, la démence du corps de Lewy, la démence vasculaire et la démence fronto-temporale.

16. Composition pharmaceutique selon la revendication 14, dans laquelle le N-[4-(1-cyclobutylpipéridin-4-yloxy)phényl]-2-(morpholin-4-yl)acétamide ou un sel de celui-ci acceptable sur le plan pharmaceutique est présent en une quantité allant de 0,1 mg à 100 mg, de préférence en une quantité allant de 0,1 mg à 60 mg, et plus préférablement en une quantité allant de 0,1 mg à 30 mg, et/ou , dans laquelle le donépézil ou un sel de celui-ci acceptable sur le plan pharmaceutique est présent en une quantité allant de 2 mg à 30 mg, et/ou dans laquelle la mémantine ou un sel de celle-ci acceptable sur le plan pharmaceutique est présente en une quantité allant de 1 mg à 40 mg.

17. Composé destiné à être utilisé selon la revendication 8, dans lequel le traitement comprend l'administration de N-[4-(1-cydobutylpipéridin-4-yloxy)phényl]-2-(morpholin-4-yl)acétamide ou d'un sel de celui-ci acceptable sur le plan pharmaceutique au patient par voie orale, nasale, locale, dermique ou parentérale.

18. Composé destiné à être utilisé selon la revendication 8, dans lequel le traitement comprend l'administration de N-[4-(1-cyclobutylpipéridin-4-yloxy)phényl]-2-(morpholin-4-yl)acétamide ou d'un sel de celui-ci acceptable sur le plan pharmaceutique au patient une à trois fois par jour, une à trois fois par semaine ou une à trois fois par mois.
